# EUROPÄISCHE PATENTSCHRIFT

(11) **EP 0 365 820 B1**
(45) Veröffentlichungstag und Bekanntmachung des Hinweises auf die Patenterteilung: **29.12.1993**
(21) Anmeldenummer: 89117264.5
(22) Anmeldetag: 19.09.1989
(51) Int. Cl.: C07D 303/48, C07D 317/34, C07D 317/72, C09K 19/58, C07D 405/12, C07D 409/12, C07D 417/12

(54) **Verwendung optisch aktiver Carbonsäureester von Sauerstoff enthaltenden Heterocyclen als Dotierstoffe in Flüssigkristallmischungen und diese enthaltende Flüssigkristallmischungen**
Utilization of optically active ester of oxygen containing heterocyclic carboxylic acids as doping agents in liquid crystalline mixtures, and those mixtures containing them
Utilisation d'esters d'acides carboxyliques hétérocycliques à oxygène optiquement actifs, comme agents dopants dans des mélanges de cristaux liquides et les mélanges de ces derniers

(30) Priorität: 24.09.1988 DE 3832502
(43) Veröffentlichungstag der Anmeldung: 02.05.1990
(73) Patentinhaber: HOECHST AKTIENGESELLSCHAFT, 65926 Frankfurt am Main (DE)
(72) Erfinder: Hemmerling, Wolfgang, Dr., D-6231 Sulzbach(Taunus) (DE); Müller, Ingrid, Dr., D-6238 Hofheim am Taunus (DE); Dübal, Hans-Rolf, Dr., D-6240 Königstein/Taunus (DE); Escher, Claus, Dr., D-6109 Mühltal (DE); Illian, Gerhard, Dr., D-6000 Frankfurt am Main 71 (DE); Murakami, Mikio, D-6240 Königstein/Taunus (DE); Ohlendorf, Dieter, Dr., D-6237 Liederbach (DE); Wingen, Rainer, Dr., D-6234 Hattersheim am Main (DE)

(56) Entgegenhaltungen:
- EP-A- 0 233 602
- EP-A- 0 263 437
- EP-A- 0 275 522
- EP-A- 0 288 813
- US-A- 4 638 073

## Beschreibung

In den prioritätsälteren, nicht-vorveröffentlichten DE-A-37 13 273 und 37 18 174 werden optisch aktive 1,3-Dioxolan-4-carbonsäureester bzw. Oxiran-2-carbonsäureester vorgestellt, die sich durch die folgende allgemeine Formel (I) beschreiben lassen:

R¹(-A¹)ⱼ(-M¹)ₖ(-A²)ₗ(-M²)ₘ(-A³)ₙ-X-Ac (I)

in der bedeuten:
R¹ -X-Ac; H;
eine geradkettige oder verzweigte Alkyl- oder Alkyloxykette mit 1-16 C-Atomen; eine geradkettige oder verzweigte Alkenyl- oder Alkenyloxykette mit 3-16 C-Atomen, wobei eine oder mehrere nicht benachbarte -CH₂-Gruppen durch -O-, -S-, -CO-, -O-CO- oder -CO-O-ersetzt sein können und wobei ein oder mehrere H-Atome durch F, Cl, Br oder CN ersetzt sein können;
oder F, Cl, Br oder CN
-A¹, -A² 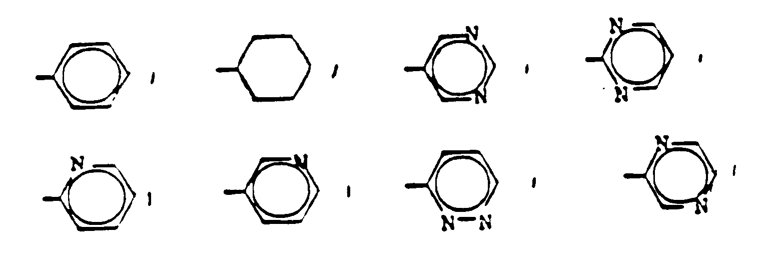 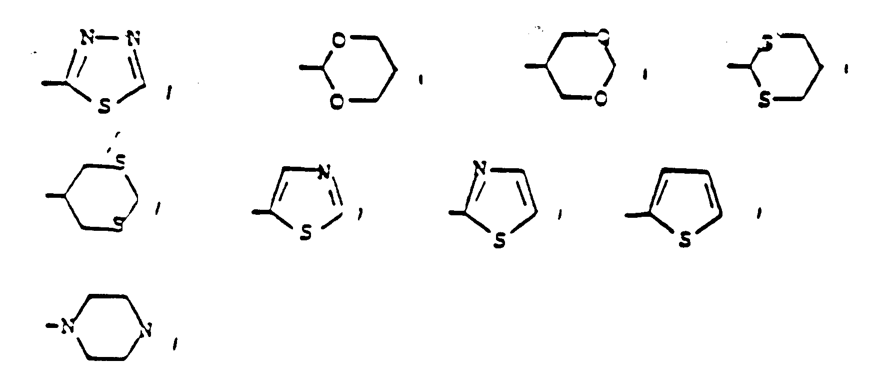
-A³ 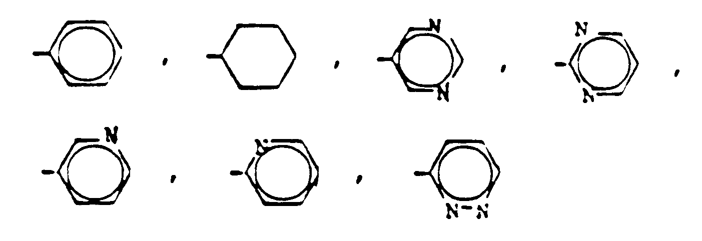
-M¹, -M² -CO-O, -O-CO, -CH₂CH₂, -CH=CH, -CH₂O, -OCH₂
j und l null, 1 oder 2
k und m null oder 1
n null, 1 oder 2 mit folgender Maßgabe: wenn j und/oder l = null sind, ist
- k =: null; wenn n = null ist, ist m = null; die Summe j+l+n ist minimal 1 und maximal 3,
- X: Sauerstoff oder Schwefel
- Ac: der Acylrest einer Oxiran-2-carbonsäure (II) 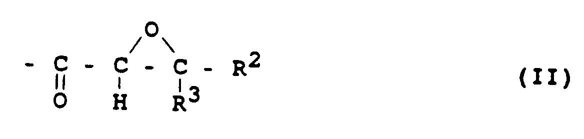 oder einer 1,3-Dioxolan-4-carbonsäure (III) 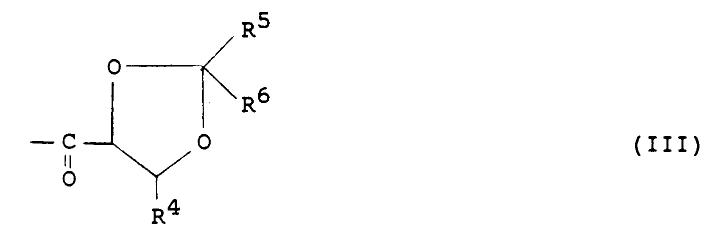
mit
- R²: H oder Alkyl mit C₁-C₁₆, oder Cyclohexyl oder Bicyclohexyl, die auch jeweils in Position 4 bzw. 4' durch eine Alkylkette mit 1 bis 16 C-Atomen substituiert sein können,
- R³: H oder Alkyl mit 1 bis 16 C-Atomen
- R⁴: H oder ein Alkylrest mit 1 bis 10 oder ein Alkenylrest mit 2 bis 10 C-Atomen
- R⁵ und R⁶: jeweils H oder ein Alkylrest mit 1 bis 10 C-Atomen, wobei ein oder mehrere H des Alkylrestes durch F ersetzt sein können, oder R² und R³ bilden zusammen mit dem C(2)-Atom des Dioxolanrings einen Cyclopentan-, Cyclohexan- oder Cycloheptanring.

Die erfindungsgemäßen optische aktiven Carbonsäureester von im endständigen Acylrest Sauerstoff enthaltenden Heterocyclen sind dadurch gekennzeichnet, daß mindestens einer der Reste -A¹, -A² und -A³ mindestens einmal durch F, Cl, Br, CN und/oder eine laterale Alkyl- oder Alkyloxygruppe mit 1 bis 10 C-Atomen substituiert ist.

Man erhält diese Verbindungen durch Umsetzung von Oxiran-2-carbonsäuren der Formel (IIa) bzw. 1,3-Dioxolan-4-carbonsäuren der Formel (IIIa) 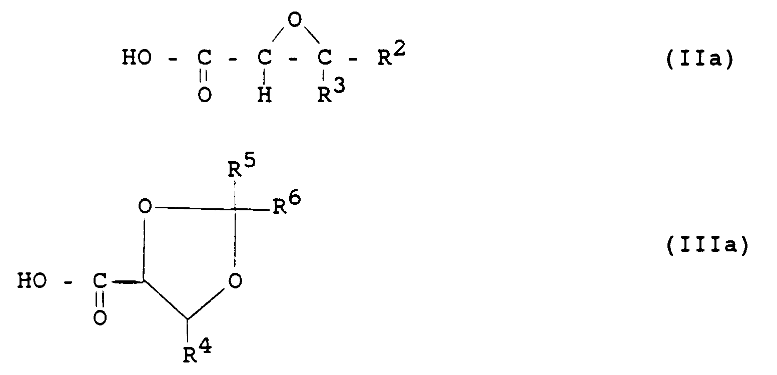
oder ihrer zur Esterbildung befähigten Derivate mit Verbindungen (IV)

R¹(-A¹)ⱼ(-M¹)ₖ(-A²)ₗ(-M²)ₘ(-A³)ₙ-XH (IV)

in an sich bekannter Weise.

Sie dienen insbesondere als Dotierstoffe zur Erzeugung ferroelektrischer Flüssigkristallmischungen.
In geneigt-smektischen Flüssigkristallphasen induzieren sie eine hohe spontane Polarisation und bewirken eine starke Schmelzpunkterniedrigung. Die erfindungsgemäßen Ester besitzen ferner ein großes negatives Δε, d.h. sie sind dielektrisch negativ.

Besonders geeignet sind optisch aktive Ester der Formel (I) bei denen
-A¹, -A², -A³ = 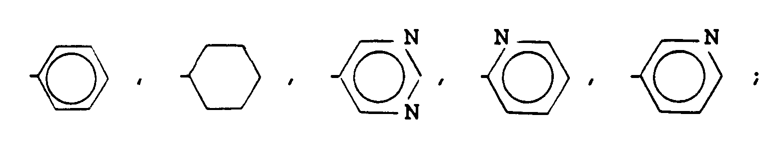
-M¹, M² =
-CO-O , -O-CO , -CH₂O , -OCH₂ ;
X = Sauerstoff
Ac = der Acylrest einer Oxiran-2-carbonsäure der Formel (II) oder einer 1,3-Dioxolan-4-carbonsäure der Formel (III) bedeuten und
R¹, R², R³, R⁴, R⁵, R⁶, j, k, l, m, n die oben genannten Bedeutungen tragen.

Die erfindungsgemäßen Flüssigkristallmischungen bestehen aus 2 bis 20, vorzugsweise 2 bis 15 Komponenten, darunter mindestens einer der erfindungsgemäß beanspruchten chiralen Dotierstoffe.

Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N-, S- oder O-haltige Heterocyclen, z.B. Pyrimidine, Zimtsäureester, Cholesterinester oder verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwarten läßt.

### Beispiel 1

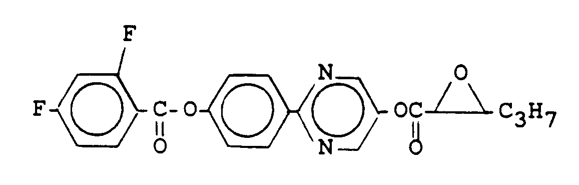

### (2R,3R)-3-Propyloxiran-2-carbonsäure[2-(4-(2,4-difluorbenzoyloxy)phenyl)pyrimidin-5-yl]ester

984 mg (3 mmol) 2,4-Difluorbenzoesäure[4-(5-hydroxypyrimidin-2-yl)phenyl]ester werden in 25 ml Dichlormethan vorgelegt. Man versetzte die gerührte Suspension mit 680 mg (3,3 mmol) Dicyclohexylcarbodiimid, 430 mg (2R,3R)-3-Propyloxiran-2-carbonsäure sowie 40 mg Dimethylaminopyridin und rührte 20 h bei Raumtemperatur weiter. Nach Chromatographie und mehrmaligem Umkristallisieren erhält man farblose Kristalle vom Schmp. 94,1 °C mit 64,2 J/g.
[α]$\frac{\text{25}}{\text{D}}$ = -14,8° (c = 2, CHCl₃)

In einer nicht-chiralen Testmischung mit der Phasenfolge K12,5S_{c}83S_{A}95N100 I weist die Verbindung bei 5 Mol-% Dotierung eine spontane Polarisation von 9 nC/cm² auf bei 60 °C.

Analog wurden synthetisiert:

### Beispiel 2

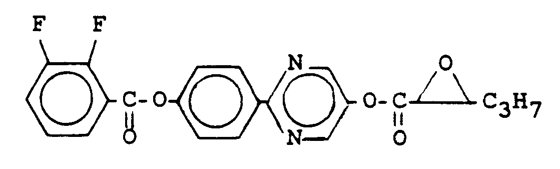

### (2R,3R)-3-Propyloxiran-2-carbonsäure[2-(4-(2,3-difluorbenzoyloxy)phenyl)pyrimidin-5-yl]ester

Schmp. 97 °C mit 70 J/g
[α]$\frac{\text{25}}{\text{D}}$ = -10° (c = 2, CHCl₃)
Spontanpolarisation: 19 nC/cm²

### Beispiel 3

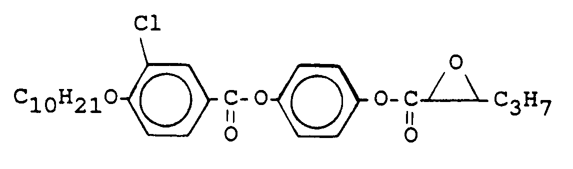

### (2R,3R)-3-Propyloxiran-2-carbonsäure[4-(3-chlor-4-decyloxybenzoyloxy)phenyl]ester

Schmp. 66,4 °C mit 61,2 J/g
[α]$\frac{\text{25}}{\text{D}}$ = -73° (c = 2, CHCl₃)
Spontanpolarisation: 14,5 nC/cm²

### Beispiel 4

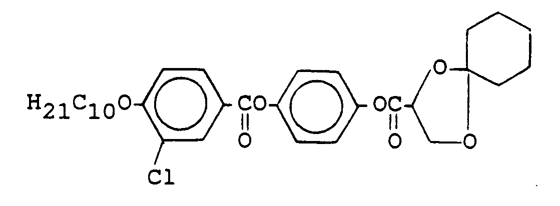

### (4R)-2,2-Pentamethylen-1,3-dioxolan-4-carbonsäure-[4-(3-chlor-4-decyloxy-benzoyloxy)]phenyl-ester

Zu einer Lösung von 4,6 g 4-[4-(3-Chlor-4-decyloxybenzoyloxy)]phenol in 50 ml Tetrahydrofuran werden 1,6 ml Triethylamin gegeben. Nach Kühlung auf 0 °C wird binnen 5 min mit einer Lösung von 2,33 g (4R)-2,2-Pentamethylen-1,3-dioxolan-4-carbonsäurechlorid in 15 ml Tetrahydrofuran versetzt. Nach 2 h wird filtriert, das Filtrat zur Trockne gebracht und chromatographisch (SiO₂, Dichlormethan/Ethylacetat 97:3) gereinigt. Eine weitere Reinigung erfolgt durch Umkristallisation aus Hexan.
Schmp. 81,3 °C mit 69,1 J/g
[α]$\frac{\text{22}}{\text{D}}$: +10,2° (c = 2,5, CH₂Cl₂)

In einer nicht-chiralen Testmischung mit der Phasenfolge K12,5S_{c}83S_{A}95_{N}100 I bei 10 Mol-% Dotierung weist die Verbindung bei 40 °C eine spontane Polarisation von 25 nC/cm² auf.

Analog werden erhalten:

### Beispiel 5

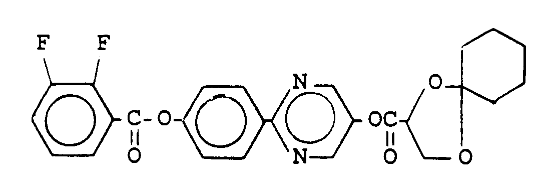

### (4R)-2,2-Pentamethylen-1,3-dioxolan-4-carbonsäure[2-(4-[2,3-difluorbenzoyloxy]phenyl)pyrimidin-5-yl]ester

Schmp. 140,7 °C mit 79,5 J/g
[α]$\frac{\text{22}}{\text{D}}$: +10,6° (c = 2,7, CH₂Cl₂)
Spontane Polarisation: 9,5 nC/cm² bei 60 °C (10 Mol-%)

### Beispiel 6

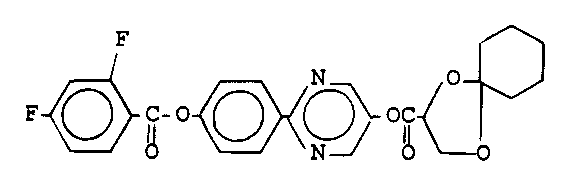

### (4R)-2,2-Pentamethylen-1,3-dioxolan-4-carbonsäure[2-(4-[2,4-difluorbenzoyloxy]phenyl)pyrimidin-5-yl]ester

Schmp. 161 °C mit 83,1 J/g
[α]$\frac{\text{22}}{\text{D}}$: +14,5° (c = 2,7, CH₂Cl₂)
Analog zu Beispiel 1 werden erhalten:

### Beispiel 7

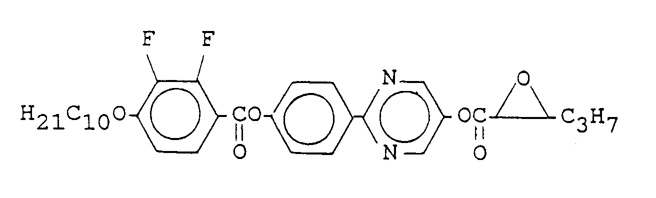

### (2R,3R)-3-Propyloxiran-2-carbonsäure-[2-(4-decyloxy-2,3-difluorbenzoyloxy)phenyl-pyrimidin-5-yl]ester

Schmp. 119 °C
[α]$\frac{\text{25}}{\text{D}}$: -12,8° (c = 2, CH₂Cl₂)

### Beispiel 8

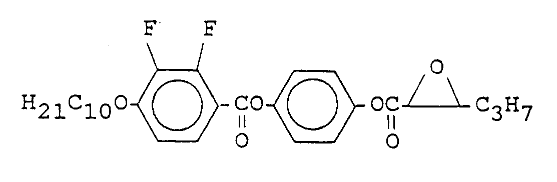

### (2R,3R)-3-Propyloxiran-2-carbonsäure-[4-(4-decyloxy-2,3-difluorbenzoyloxy)phenyl]ester

[α]$\frac{\text{25}}{\text{D}}$: -12,9° (c = 2, CH₂Cl₂)
Phasenfolge: K 55 I

Analog zu Beispiel 4 werden erhalten:

### Beispiel 9

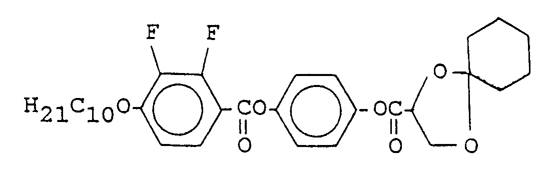

### (4R)-2,2-Pentamethylen-1,3-dioxolan-4-carbonsäure[4-(4-decyloxy-2,3-difluor-benzoyloxy)phenyl]ester

Schmp. 86,7 °C
[α]$\frac{\text{25}}{\text{D}}$: +10,6° (c = 2,5, CH₂Cl₂)

### Beispiel 10

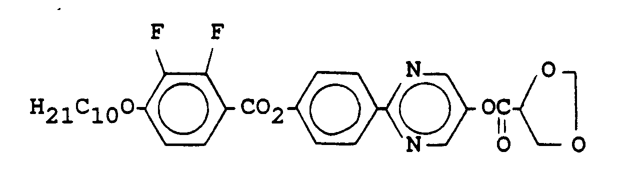

### (4R)-2,2-Pentamethylen-1,3-dioxolan-4-carbonsäure-[2-(4-decyloxy-2,3-difluor-benzoyloxy)phenyl-pyrimidin-5-yl]ester

[α]$\frac{\text{25}}{\text{D}}$: -12,8° (c = 2, CH₂Cl₂)
Phasenfolge K(104Sₓ133) 149 N* 162 I

### Anwendungsbeispiel 1

a) Eine ferroelektrische flüssigkristalline Mischung besteht aus 6 Komponenten

| | |
|---|---|
| 5-Octyloxy-2-(4-butyloxy-phenyl)-pyrimidin | 25,4 Mol-% |
| 5-Octyloxy-2-(4-hexyloxy-phenyl)-pyrimidin | 24,1 Mol-% |
| 5-Octyloxy-2-(4-octyloxy-phenyl)-pyrimidin | 11,1 Mol-% |
| 5-Octyloxy-2-(4-decyloxy-phenyl)-pyrimidin | 20,2 Mol-% |
| trans-4-Pentyl-cyclohexancarbonsäure-[4-(5-decyl-pyrimidin-2-yl)]-phenylester | 14,2 Mol-% |
| (2R,3R)-3-Propyloxiran-2-carbonsäure-[2-(4-decyloxy-2,3-difluorbenzoyloxy)-phenyl-pyrimidin-5-yl]ester | 5 Mol-% |

zeigt folgende flüssigkristalline Phasenbereiche:
S$\frac{\text{*}}{\text{c}}$ 81 N* 103 I
und weist bei 40 °C eine spontane Polarisation von 36 nC·cm⁻² und eine Schaltzeit von 42,6 µs auf.
b) Im Vergleich dazu weist die, in DE 38 31 226.3 beanspruchte flüssigkristalline Mischung, die sich von der obengenannten ferroelektrischen Mischung nur dadurch unterscheidet, daß sie keinen Dotierstoff enthält, folgende Phasenbereiche auf
S_{c} 84 S_{A} 93 N 105 I

### Anwendungsbeispiel 2

a) Eine ferroelektrische flüssigkristalline Mischung besteht aus 6 Komponenten

| | |
|---|---|
| 5-Octyloxy-2-(4-butyloxy-phenyl)-pyrimidin | 24 Mol-% |
| 5-Octyloxy-2-(4-hexyloxy-phenyl)-pyrimidin | 22,8 Mol-% |
| 5-Octyloxy-2-(4-octyloxy-phenyl)-pyrimidin | 10,5 Mol-% |
| 5-Octyloxy-2-(4-decyloxy-phenyl)-pyrimidin | 19,2 Mol-% |
| trans-4-Pentyl-cyclohexancarbonsäure-[4-(5-decyl-pyrimidin-2-yl)]-phenylester | 13,5 Mol-% |
| (2R,3R)-3-Propyloxiran-2-carbonsäure-[4-(4-decyloxy-2,3-difluorbenzoyloxy)-phenyl]ester | 10 Mol-% |

zeigt folgende flüssigkristalline Phasenbereiche:
S$\frac{\text{*}}{\text{c}}$ 73 S$\frac{\text{*}}{\text{A}}$ 81 N* 94 I
und weist bei 40 °C eine spontane Polarisation von 38 nC·cm⁻² und eine Schaltzeit von 23,2 µs auf.
b) Im Vergleich dazu weist die, in DE-A-38 31 226.3 beanspruchte flüssigkristalline Mischung, die sich von der obengenannten ferroelektrischen Mischung nur dadurch unterscheidet, daß sie keinen Dotierstoff enthält, folgende Phasenbereiche auf
S_{c} 84 S_{A} 93 N 105 I

## Patentansprüche

1. Verwendung von Verbindungen der allgemeinen Formel (I)
R¹(-A¹)ⱼ(-M¹)ₖ(-A²)ₗ(-M²)ₘ(-A³) ₙ-X-Ac (I)
in der bedeuten
R¹ -X-Ac; H;
eine geradkettige oder verzweigte Alkyl- oder Alkyloxykette mit 1-16 C-Atomen; eine geradkettige oder verzweigte Alkenyl- oder Alkenyloxykette mit 3-16 C-Atomen, wobei eine oder mehrere nicht benachbarte -CH₂-Gruppen durch -O-, -S-, -CO-, -O-CO- oder -CO-O-ersetzt sein können und wobei ein oder mehrere H-Atome durch F, Cl, Br oder CN ersetzt sein können; oder F, Cl, Br oder CN
-A¹, -A² 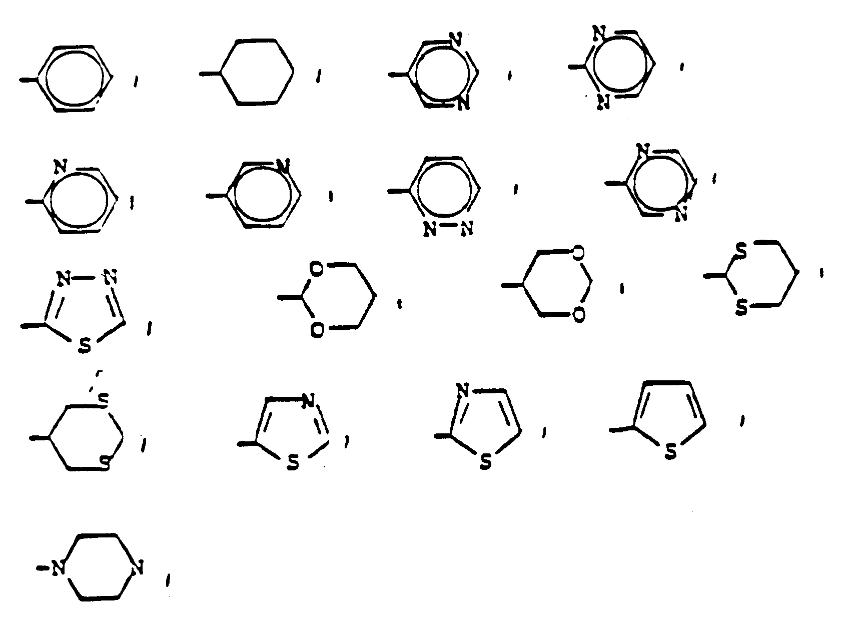
-A³ 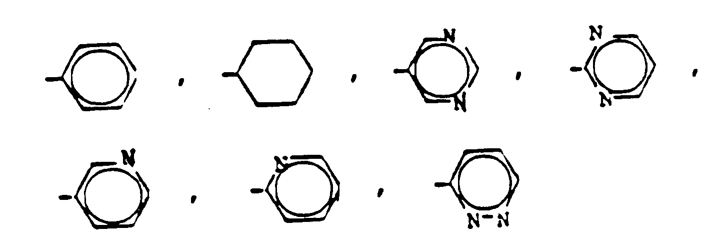
-M¹, -M² -CO-O, -O-CO, -CH₂CH₂, -CH=CH, -CH₂O, -OCH₂
j und l null, 1 oder 2
k und m null oder 1
n null, 1 oder 2
mit folgender Maßgabe: wenn j und/oder l = null sind, ist k = null; wenn n = null ist, ist m = null; die Summe j+l+n ist minimal 1 und maximal 3,
X Sauerstoff oder Schwefel
Ac der Acylrest einer Oxiran-2-carbonsäure (II) 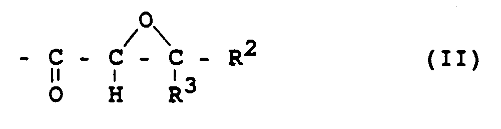
oder einer 1,3-Dioxolan-4-carbonsäure (III) 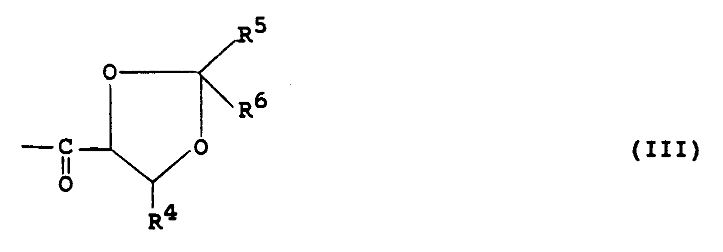 mit
R² H oder Alkyl mit C₁-C₁₆, oder Cyclohexyl oder Bicyclohexyl, die auch jeweils in Position 4 bzw. 4' durch eine Alkylkette mit 1 bis 16 C-Atomen substituiert sein können,
R³ H oder Alkyl mit 1 bis 16 C-Atomen
R⁴ H oder ein Alkylrest mit 1 bis 10 oder ein Alkenylrest mit 2 bis 10 C-Atomen
R⁵ und R⁶ jeweils H oder ein Alkylrest mit 1 bis 10 C-Atomen, wobei ein oder mehrere H des Alkylrestes, durch F ersetzt sein können, oder R² und R³ bilden zusammen mit dem C(2)-Atom des Dioxolanrings einen Cyclopentan-, Cyclohexan- oder Cycloheptanring,
mit der Maßgabe, daß mindestens einer der Reste -A¹, -A² und -A³ mindestens einmal durch F, Cl, Br, CN und/oder eine laterale Alkyl- oder Alkyloxygruppe mit 1 bis 10 C-Atomen substituiert ist, als Dotierstoffe in Flüssigkristallmischungen.

2. Verwendung von optisch aktiven Verbindungen der Formel (I), wobei die Symbole folgende Bedeutungen haben:
-A¹, -A², -A³ = 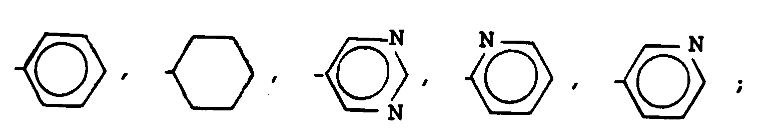
-M¹, M² =
-CO-O , -O-CO , -CH₂O , -OCH₂ ;
X = Sauerstoff
Ac = der Acylrest einer Oxiran-2-carbonsäure der Formel (II) oder einer 1,3-Dioxolan-4-carbonsäure der Formel (III)
und R¹, R², R³, R⁴, R⁵, R⁶, j, k, l, m, n die in Anspruch 1 genannten Bedeutungen tragen.

3. Verwendung von optisch aktiven Verbindungen der Formel (I) nach Anspruch 2, dadurch gekennzeichnet, daß Ac der Acylrest einer Oxiran-2-carbonsäure der Formel (II) ist.

4. Verwendung von optisch aktiven Verbindung en der Formel (I) nach Anspruch 2, dadurch gekennzeichnet, daß Ac der Acylrest einer 1,3-Dioxolan-4-carbonsäure der Formel (III) ist.

5. Flüssigkristallmischung enthaltend mindestens einen Ester der Formel (I) nach Anspruch 1.

6. Flüssigkristallmischung enthaltend mindestens einen Ester der Formel (I) nach Anspruch 2.

7. Optisch aktive Verbindung der Formel (I) nach Anspruch 1.

8. Optisch aktive Verbindung der Formel (I) nach Anspruch 2.

9. Optisch aktive Verbindung der Formel (I) nach Anspruch 3.

10. Optisch aktive Verbindung der Formel (I) nach Anspruch 4.

## Claims

1. Use of a compound of the general formula (I)
R¹(-A¹)ⱼ(-M¹)ₖ(-A²)ₗ(-M²)ₘ(-A³)ₙ-X-Ac (I)
in which
R¹ is -X-Ac; H;
a straight-chain or branched alkyl or alkyloxy chain having 1-16 carbon atoms; a straight-chain or branched alkenyl or alkenyloxy chain having 3-16 carbon atoms, it being possible for one or more non-adjacent -CH₂- groups to be replaced by -O-, -S-, -CO-, -O-CO- or -CO-O- and one or more hydrogen atoms to be replaced by F, Cl, Br or CN; or R¹ is F, Cl, Br or CN,
-A¹, -A² are 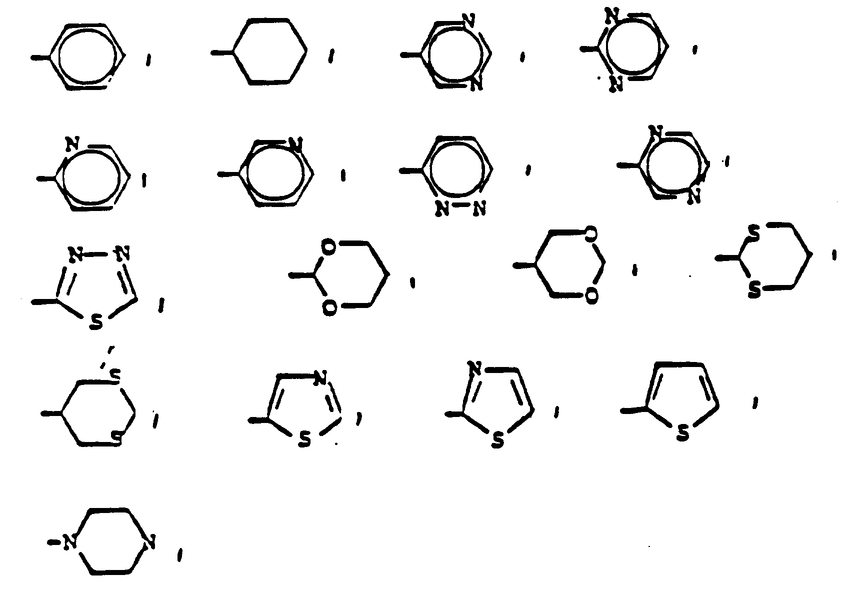
-A³ is 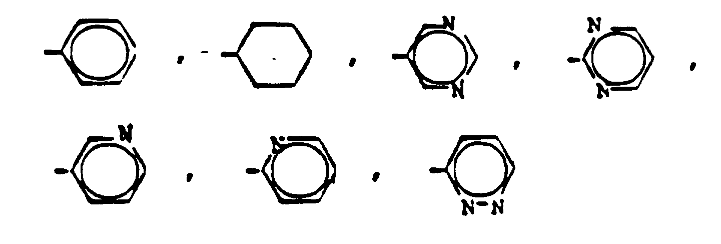
-M¹, -M² are -CO-O, -O-CO, -CH₂CH₂, -CH=CH, -CH₂O, -OCH₂,
j and l are zero, 1 or 2,
k and m are zero or 1,
n is zero, 1 or 2,
with the following proviso: if j and/or l are zero,
k is zero; if n is zero, m is zero; the sum of j+l+n is at least 1 and at most 3,
X is oxygen or sulfur,
Ac is the acyl radical of an oxirane-2-carboxylic acid (II) 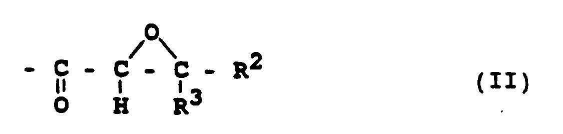
or of a 1,3-dioxolane-4-carboxylic acid (III) 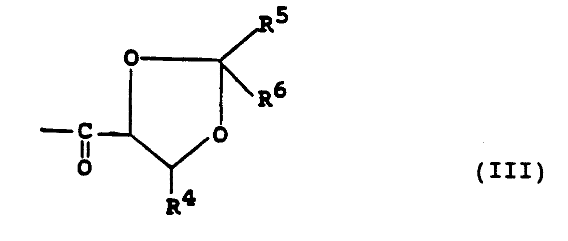 where
R² is H or C₁-C₁₆-alkyl or cyclohexyl or bicyclohexyl, each of which can also be substituted in the 4- or 4'-position by an alkyl chain of 1 to 16 carbon atoms,
R³ is H or alkyl having 1 to 16 carbon atoms,
R⁴ is H or an alkyl radical having 1 to 10, or an alkenyl radical having 2 to 10, carbon atoms,
R⁵ and R⁶ are each H or an alkyl radical having 1 to 10 carbon atoms, in which one or more hydrogen atoms of the alkyl radical can be replaced by F, or R² and R³ together with the C (2) atom of the dioxolane ring form a cyclopentane, cyclohexane or cycloheptane ring, with the proviso that at least one of the radicals -A¹, -A² and -A³ is at least monosubstituted by F, Cl, Br, CN and/or a lateral alkyl or alkyloxy group having 1 to 10 carbon atoms, as doping substances in liquid crystal mixtures.

2. Use of optically active compounds of the formula (I) in which the symbols have the following meanings:
-A¹, -A², -A³ are 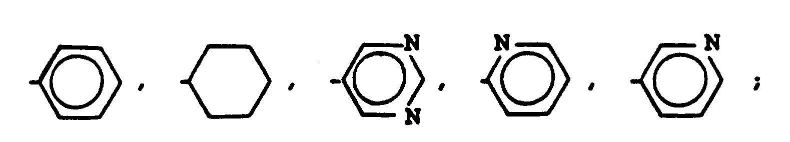
-M¹, M² are
-CO-O , -O-CO , -CH₂O , -OCH₂ ,
X is oxygen,
Ac is the acyl radical of an oxirane-2-carboxylic acid of the formula (II) or of a 1,3-dioxolane-4-carboxylic acid of the formula (III)
and R¹, R², R³, R⁴, R⁵, R⁶, j, k, l, m, n have the meanings mentioned in claim 1.

3. Use of optically active compounds of the formula (I) as claimed in claim 2, wherein Ac is the acyl radical of an oxirane-2-carboxylic acid of the formula (II).

4. Use of optically active compounds of the formula (I) as claimed in claim 2, wherein Ac is the acyl radical of a 1,3-dioxolane-4-carboxylic acid of the formula (III).

5. A liquid crystal mixture containing at least one ester of the formula (I) as claimed in claim 1.

6. A liquid crystal mixture containing at least one ester of the formula (I) as claimed in claim 2.

7. An optically active compound of the formula (I) as claimed in claim 1.

8. An optically active compound of the formula (I) as claimed in claim 2.

9. An optically active compound of the formula (I) as claimed in claim 3.

10. An optically active compound of the formula (I) as claimed in claim 4.

## Revendications

1. Utilisation de composés répondant à la formule générale (I)
R¹(A¹)ⱼ(-M¹)ₖ(-A²)ₗ(-M²)ₘ(-A³)ₙ-X-Ac (I)
(dans laquelle, R¹ représente -X-Ac; H;
une chaîne alkyle ou alkyloxy, linéaire ou ramifiée, ayant 1 à 16 atomes de carbone, une chaîne alcényle ou alcényloxy linéaire ou ramifiée ayant 3 à 16 atomes de carbone, un ou plusieurs groupes -CH₂- non voisins pouvant être remplacés par -O-, -S-, -CO- -O-CO-, ou -CO-O-et un ou plusieurs atomes de H pouvant être remplacés par F, Cl, Br ou CN;
ou bien R¹ représente F, Cl, Br, ou CN;
-A¹, A² représentent chacun : 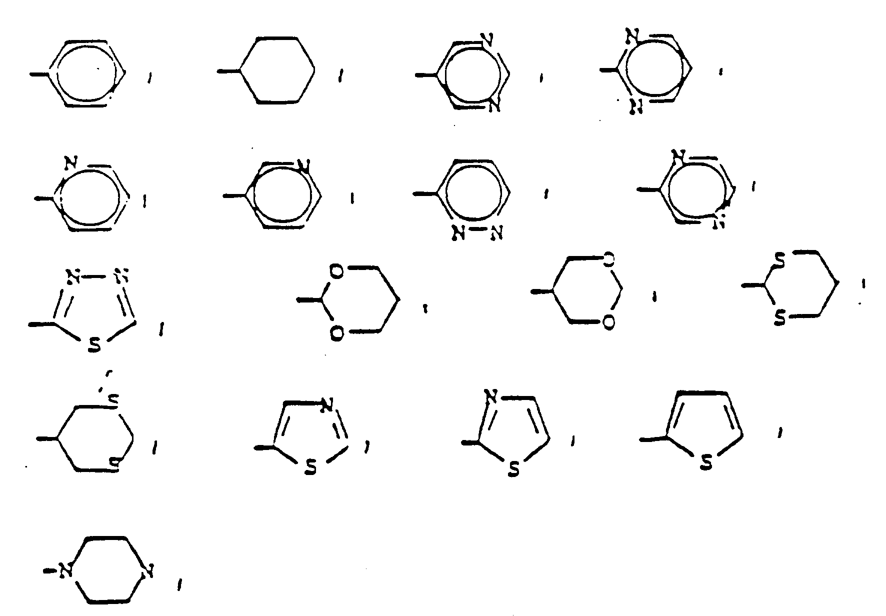 A³ représente 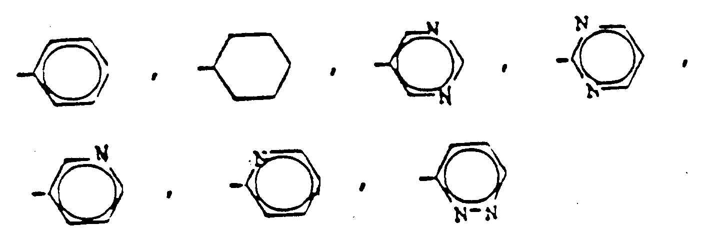 -M¹, -M² représentent chacun
-CO-O, -O-CO, -CH₂CH₂, -CH=CH, -CH₂O, -OCH₂
j et l sont des nombres valant 0, 1 ou 2.
k et m sont des nombres valant chacun 0 ou 1,
n est nul ou vaut 1, ou 2,
à la condition que, lorsque j et/ou l est ou sont nul(s), k est nul; lorsque n est nul, m est nul; la somme (j+l+n) vaut au minimum 1 et au maximum 3;
X représente un atome d'oxygène ou de soufre,
Ac représente le reste acyle d'un acide oxiranne-2-carboxylique (II) 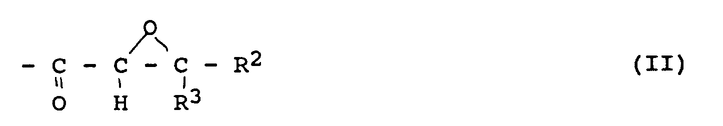 ou d'un acide 1,3-dioxolanne-4-carboxylique (III) 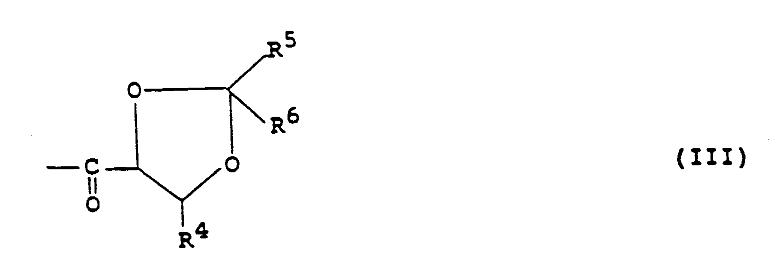 formules dans lesquelles ,
R² représente H ou un groupe alkyle ayant 1 à 16 atomes de carbone ou un groupe cyclohexyle ou bicyclohexyle, qui peuvent également être chacun substitués en position 4 ou 4' par une chaîne alkyle ayant 1 à 16 atomes de carbone,
R³ représente H ou un groupe alkyle ayant 1 à 16 atomes de carbone,
R⁴ représente H ou un reste alkyle ayant 1 à 10 ou un reste alcényle ayant 2 à 10 atomes de carbone,
R⁵ et R⁶ représentent chacun H ou un reste alkyle ayant 1 à 10 atomes de carbone, un ou plusieurs des atomes de H du reste alkyle pouvant être remplacé(s) par F, ou bien R² et R³ forment, avec l'atome C(2) du noyau dioxolanne, un noyau de cyclopentane, de cyclohexane ou de cycloheptane,
à la condition qu'au moins l'un des restes -A¹, -A² et -A³ soit substitué au moins une fois par F, Cl, Br, CN et/ou par un groupe latéral alkyle ou alkyloxy ayant 1 à 10 atomes de carbone), comme substances de dopage dans des mélanges à propriétés de cristaux liquides.

2. Utilisation de composés optiquement actifs de formule (I), dans laquelle les symboles ont les sens suivants :
-A¹, -A², -A³ , 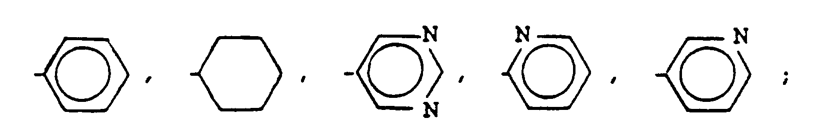 -M¹, M² =
-CO-O, -O-CO, -CH₂O, -OCH₂,
X représente un atome d'oxygène,
Ac représente le reste acyle d'un acide oxiranne-2-carboxylique de formule (II) ou d'un acide 1,3-dioxolanne-4-carboxylique de formule (III), et R¹, R², R³, R⁴, R⁵, R⁶, j, k, l, m, n, ont les sens indiqués à la revendication 1.

3. Utilisation de composés optiquement actifs de formule (I) selon la revendication 2, caractérisée en ce que Ac représente le reste acyle d'un acide oxiranne-2-carboxylique de formule (II).

4. Utilisation de composés optiquement actifs de formule (I) selon la revendication 2, caractérisée en ce que Ac représente le reste acyle d'un acide 1,3-dioxolanne-4-carboxylique de formule (III).

5. Mélange à propriétés de cristaux liquides, contenant au moins un ester de formule (I) selon la revendication 1.

6. Mélange à propriétés de cristaux liquides, contenant au moins un ester de formule (I) selon la revendication 2.

7. Composé optiquement actif de formule (I) selon la revendication 1.

8. Composé optiquement actif de formule (I) selon la revendication 2.

9. Composé optiquement actif de formule (I) selon la revendication 3.

10. Composé optiquement actif de formule (I) selon la revendication 4.
